(19) Europäisches Patentamt
European Patent Office
Office européen des brevets

(11) EP 0 988 021 B1

(12) # FASCICULE DE BREVET EUROPEEN

(45) Date de publication et mention de la délivrance du brevet:
**12.11.2003 Bulletin 2003/46**

(21) Numéro de dépôt: **98924391.0**

(22) Date de dépôt: **06.05.1998**

(51) Int Cl.[7]: **A61K 7/13**

(86) Numéro de dépôt international:
**PCT/FR98/00913**

(87) Numéro de publication internationale:
**WO 98/055083 (10.12.1998 Gazette 1998/49)**

(54) **COMPOSITION DE TEINTURE D'OXYDATION DES FIBRES KERATINIQUES ET PROCEDE DE TEINTURE METTANT EN OEUVRE CETTE COMPOSITION**

OXYDATIONSFÄRBEMITTEL FÜR KERATINFASERN UND FÄRBEVERFAHREN UNTER VERWENDUNG DIESER ZUSAMMENSETZUNG

KERATIN FIBRE OXIDATION DYEING COMPOSITION AND DYEING METHOD USING SAME

(84) Etats contractants désignés:
**AT BE CH CY DE DK ES FI FR GB GR IE IT LI LU MC NL PT SE**

(30) Priorité: **03.06.1997 FR 9706802**

(43) Date de publication de la demande:
**29.03.2000 Bulletin 2000/13**

(73) Titulaire: **L'OREAL**
**75008 Paris (FR)**

(72) Inventeur: **MAUBRU, Mireille**
**F-78400 Chatou (FR)**

(74) Mandataire: **Goulard, Sophie**
**L'OREAL,**
**DPI,**
**6, rue Sincholle**
**92585 Clichy Cedex (FR)**

(56) Documents cités:
**EP-A- 0 310 675** **EP-A- 0 716 846**
**WO-A-94/00100** **WO-A-97/19999**

- **YOSHIO TSUJINO ET AL.: "Hair coloring and waving using oxidases." J. SOC. COSMET. CHEM., vol. 42, juillet 1991 - août 1991, NEW YORK, pages 273-282, XP002055535**
- **MASAHIRO AOKI ET AL.: "Significance of Uricase in Oxidase-Induced Coloring Reaction of p-Phenylenediamine." JOURNAL OF ORGANIC CHEMISTRY, vol. 61, 1996, EASTON, PA, pages 5610-5616, XP002055536**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 988 021 B1

Printed by Jouve, 75001 PARIS (FR)

## Description

L'invention a pour objet une composition pour la teinture d'oxydation des fibres kératiniques, et en particulier des fibres kératiniques humaines telles que les cheveux comprenant, dans un milieu approprié pour la teinture, au moins un colorant d'oxydation hétérocyclique, au moins une enzyme de type oxydo-réductase à 2 électrons en présence d'au moins un donneur pour ladite enzyme, ainsi que le procédé de teinture mettant en oeuvre cette composition.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des bases hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

La coloration d'oxydation des fibres kératiniques est généralement réalisée en milieu alcalin, en présence de peroxyde d'hydrogène. Toutefois, l'utilisation des milieux alcalins en présence de peroxyde d'hydrogène présentent pour inconvénient d'entraîner une dégradation non négligeable des fibres, ainsi qu'une décoloration des fibres kératiniques qui n'est pas toujours souhaitable.

La coloration d'oxydation des fibres kératiniques peut également être réalisée à l'aide de systèmes oxydants différents du peroxyde d'hydrogène tels que des systèmes enzymatiques. Ainsi il a déjà été proposé de teindre les fibres kératiniques, notamment dans la demande de brevet EP-A-0 310 675, avec des compositions comprenant un précurseur de colorant d'oxydation de type benzénique, en association avec des enzymes telles que la pyranose-oxydase, la glucose-oxydase ou bien l'uricase, en présence d'un donneur pour lesdites enzymes. Il est également connu d'utiliser des composés pyridiniques monocycliques en association avec un systeme oxydant enzymatique pour la coloration d'oxydation des fibres kératiniques, notamment dans les demandes de brevet européen EP 0 716 846 et EP 0 310 675 où la 2,6-diamino-pyridine est utilisée en association avec une enzyme de type oxydo-réductase à 2 électrons et un donneur pour ladite enzyme. Ces procédés de teinture, bien qu'étant mis en oeuvre dans des conditions n'entraînant pas une dégradation des fibres kératiniques comparable à celle engendrée par les teintures réalisées en présence de peroxyde d'hydrogène, conduisent à des colorations moins puissantes.

Or, la demanderesse vient maintenant de découvrir qu'il est possible d'obtenir de nouvelles teintures, capables de conduire à des colorations puissantes sans engendrer de dégradation significative, ni de décoloration des fibres kératiniques, peu sélectives et résistant bien aux diverses agressions que peuvent subir les cheveux, en associant au moins une base d'oxydation, au moins un coupleur, et au moins une enzyme de type oxydo-réductase à 2 électrons en présence d'au moins un donneur pour ladite enzyme, la ou les bases d'oxydation et/ou le ou les coupleurs utilisés étant choisis parmi des composés hétérocycliques convenablement sélectionnés.

Cette découverte est à la base de la présente invention.

L'invention a donc pour premier objet une composition prête à l'emploi, pour la teinture d'oxydation des fibres kératiniques et en particulier des fibres kératiniques humaines telles que les cheveux, comprenant l'association d'au moins une base d'oxydation et d'au moins un coupleur, caractérisée par le fait qu'elle comprend, dans un milieu approprié pour la teinture, au moins une enzyme de type oxydo-réductase à 2 électrons en présence d'au moins un donneur pour ladite enzyme, et que ladite base d'oxydation et/ou ledit coupleur est choisi parmi les composés hétérocycliques à l'exclusion des composés pyridiniques monocycliques.

La composition tinctoriale prête à l'emploi conforme à l'invention conduit à des colorations plus puissantes que celles obtenues avec des compositions tinctoriales contenant uniquement des colorants benzéniques en présence d'une enzyme de type oxydo-réductase à 2 électrons, et d'un donneur pour ladite enzyme, ce qui n'est pas le cas avec un système oxydant classique tel que le peroxyde d'hydrogène. Les colorations obtenues avec la composition tinctoriale prête à l'emploi conforme à l'invention présentent par ailleurs une faible sélectivité et d'excellentes propriétés de résistances à la fois vis à vis des agents atmosphériques tels que la lumière et les intempéries et vis à vis de la

transpiration et des différents traitements que peuvent subir les cheveux (lavages, déformations permanentes).

L'invention a également pour objet un procédé de teinture d'oxydation des fibres kératiniques mettant en oeuvre cette composition tinctoriale prête à l'emploi.

La ou les oxydo-réductases à 2 électrons utilisées dans la composition tinctoriale prête à l'emploi conforme à l'invention peuvent notamment être choisies parmi les pyranose oxydases, les glucose oxydases, les glycérol oxydases, les lactates oxydases, les pyruvate oxydases, et les uricases.

Selon l'invention, l'oxydo-réductase à 2 électrons est de préférence choisie parmi les uricases d'origine animale, microbiologique ou biotechnologique.

A titre d'exemple, on peut notamment citer l'uricase extraite de foie de sanglier, l'uricase d'Arthrobacter globiformis, ainsi que l'uricase d'Aspergillus flavus.

La ou les oxydo-réductases à 2 électrons peuvent être utilisées sous forme cristalline pure ou sous une forme diluée dans un diluant inerte pour ladite oxydo-réductase à 2 électrons.

La ou les oxydo-réductases à 2 électrons conformes à l'invention représentent de préférence de 0,01 à 20 % en poids environ du poids total de la composition tinctoriale prête à l'emploi, et encore plus préférentiellement de 0,1 à 5 % en poids environ de ce poids.

Selon l'invention, on entend par donneur, les différents substrats également nécessaires au fonctionnement de ladite ou desdites oxydo-réductases à 2 électrons.

La nature du donneur (ou substrat) pour ladite enzyme varie en fonction de la nature de l'oxydo-réductase à 2 électrons qui est utilisée. Par exemple, à titre de donneur pour les pyranose oxydases, on peut citer le D-glucose, le L-sorbose et le D-xylose ; à titre de donneur pour les glucose oxydases, on peut citer le D-glucose, à titre de donneur pour les glycérol oxydases, on peut citer le glycérol et la dihydroxyacétone ; à titre de donneur pour les lactate oxydases, on peut citer l'acide lactique et ses sels ; à titre de donneur pour les pyruvate oxydases, on peut citer l'acide pyruvique et ses sels ; et enfin à titre de donneur pour les uricases, on peut citer l'acide urique et ses sels.

Le ou les donneurs (ou substrats) utilisés conformément à l'invention représentent de préférence de 0,01 à 20 % en poids environ du poids total de la composition tinctoriale prête à l'emploi conforme à l'invention et encore plus préférentiellement de 0,1 à 5 % en environ de ce poids.

Parmi les bases d'oxydation hétérocycliques utilisables dans la composition tinctoriale prête à l'emploi selon l'invention, on peut notamment citer les dérivés pyrimidiniques, les dérivés pyrazoliques, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-106 059, comme la 2,4,5,6-tétra-aminopyrimidine, la 4-hydroxy 2,5,6-triaminopyrimidine, et leurs sels d'addition avec un acide, ainsi que les dérivés pyrazolopyrimidiniques tels que la pyrazolo-[1,5-a]-pyrimidine-3,7-diamine, la 2-méthyl pyrazolo-[1,5-a]-pyrimidine-3,7-diamine, la 2,5-diméthyl pyrazolo-[1,5-a]-pyrimidine-3,7-diamine, la pyrazolo-[1,5-a]-pyrimidine-3,5-diamine, la 2,7-diméthyl pyrazolo-[1,5-a]-pyrimidine-3,5-diamine, le 3-amino pyrazolo-[1,5-a]-pyrimidin-7-ol, le 3-amino 5-méthyl pyrazolo-[1,5-a]-pyrimidin-7-ol, le 3-amino pyrazolo-[1,5-a]-pyrimidin-5-ol, le 2-(3-amino pyrazolo-[1,5-a]-pyrimidin-7-ylamino)-éthanol, la 3-amino-7-β-hydroxyéthylamino-5-méthyl-pyrazolo-[1,5-a]-pyrimidine, le 2-(7-amino pyrazolo-[1,5-a]-pyrimidin-3-ylamino)-éthanol, le 2-[(3-amino-pyrazolo-[1,5-a]-pyrimidin-7-yl)-(2-hydroxyéthyl)-amino]-éthanol, le 2-[(7-amino-pyrazolo-[1,5-a]-pyrimidin-3-yl)-(2-hydroxyéthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N-7, N-7-tetraméthyl pyrazolo-[1,5-a]-pyrimidine-3,7-diamine, et leurs sels d'addition et leurs formes tautomères, lorsqu'il existe un équilibre tautomérique.

Parmi les dérivés pyrazoliques, on peut plus particulièrement citer les composés décrits dans les brevets ou demandes de brevet DE 3 843 892, DE 4 133 957, DE 4 234 886, DE 4 234 887, FR 2 733 749, FR 2 735 685, WO 94/08969 et WO 94/08970, comme le 4,5-diamino pyrazole, le 4,5-diamino 1-méthyl pyrazole, le 1-benzyl 4,5-diamino pyrazole, le 3,4-diamino pyrazole, le 1-benzyl 4,5-diamino 3-méthyl pyrazole, le 4-amino 1,3-diméthyl 5-hydrazino pyrazole, le 4,5-diamino 3-méthyl 1-phényl pyrazole, le 4,5-diamino 3-méthyl 1-tert-butyl pyrazole, le 4,5-diamino 1-méthyl 3-tert-butyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, et leurs sels d'addition avec un acide.

Lorsqu'elles sont présentes, la ou les bases d'oxydation hétérocycliques conformes à l'invention représentent de préférence de 0,0005 à 12 % en poids environ du poids total de la composition tinctoriale prête à l'emploi, et encore plus préférentiellement de 0,005 à 6 % en poids environ de ce poids.

Parmi les coupleurs hétérocycliques utilisables dans la composition tinctoriale prête à l'emploi selon l'invention, on peut notamment citer les dérivés indoliques, les dérivés indoliniques, les dérivés de benzimidazole, les dérivés de benzomorpholine, les dérivés de sésamol, les dérivés pyrazolo-azoliques, les dérivés pyrrolo-azoliques, les dérivés imidazolo-azoliques, les dérivés pyrazolo-pyrimidiniques, les dérivés de pyrazolin-3,5-diones, les dérivés pyrrolo-[3,2-d]-oxazoliques, les dérivés pyrazolo-[3,4-d]-thiazoliques, les dérivés S-oxyde-thiazolo-azoliques, les dérivés S,S-dioxyde-thiazolo-azoliques, et leurs sels d'addition avec un acide.

Parmi les dérivés indoliques utilisables à titre de coupleurs hétérocycliques dans la composition tinctoriale conforme à l'invention, on plus particulièrement citer les composés de formule (I) suivante, et leurs sels d'addition avec un acide :

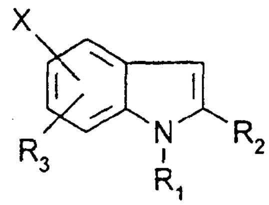
(I)

dans laquelle :

$R_1$ représente un atome d'hydrogène, un radical alkyle en $C_1$-$C_4$, un radical mono- ou polyhydroxyalkyle en $C_2$-$C_4$ ou aminoalkyle en $C_1$-$C_4$ dont l'amine est mono ou disubstituée par un groupement alkyle en $C_1$-$C_4$ ;
$R_2$ représente un atome d'hydrogène ou un radical alkyle en $C_1$-$C_4$ ;
$R_3$ représente un atome d'hydrogène, un radical alkyle en $C_1$-$C_4$ ou hydroxyle ;
X représente un radical hydroxyle ou $NHR_4$ dans lequel $R_4$ représente un atome d'hydrogène, un radical alkyle en $C_1$-$C_4$ ou hydroxyalkyle en $C_1$-$C_4$.

Parmi les composés indoliques de formule (I) ci-dessus, on peut plus particulièrement citer le 4-hydroxy indole, le 6-hydroxy indole, le 7-amino indole, le 6-amino indole, le 7-hydroxy indole, le 7-éthyl 6-(β-hydroxyéthyl)amino indole, le 4-amino indole, le 6-hydroxy 1-méthyl indole, le 5,6-dihydroxy indole, le 4-hydroxy 1-N-méthyl indole, le 4-hydroxy 2-méthyl indole, le 4-hydroxy 5-méthyl indole, le 4-hydroxy 1-N-(β-hydroxyéthyl) indole, le 4-hydroxy 1-N-(β-hydroxypropyl) indole, le 1-N-(β,γ-dihydroxypropyl) 4-hydroxy indole, le 4-hydroxy 1-N-(β-hydroxyethyl) 5-méthyl indole, le 1-N-(γ-diméthylaminopropyl) 4-hydroxy indole, et leur sels d'addition avec un acide.

Parmi les dérivés indoliniques utilisables à titre de coupleurs hétérocycliques dans la composition tinctoriale conforme à l'invention, on peut particulièrement citer la 4-hydroxy indoline, la 6-hydroxy indoline, la 6-amino indoline, la 5,6-dihydroxy indoline, et leurs sels d'addition avec un acide.

Parmi les dérivés de benzimidazole utilisables à titre de coupleurs hétérocycliques dans la composition tinctoriale conforme à l'invention, on peut plus particulièrement citer les composés de formule (II) suivante, et leurs sels d'addition avec un acide :

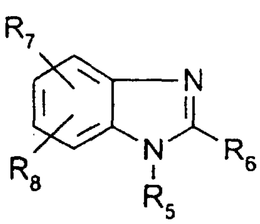
(II)

dans laquelle :

$R_5$ représente un atome d'hydrogène ou un radical alkyle en $C_1$-$C_4$,
$R_6$ représente un atome d'hydrogène, un radical alkyle en $C_1$-$C_4$ ou phényle,
$R_7$ représente un radical hydroxyle, amino ou méthoxy,
$R_8$ représente un atome d'hydrogène, un radical hydroxyle, méthoxy ou alkyle en $C_1$-$C_4$ ;

sous réserve que :

- lorsque $R_7$ désigne un radical amino, alors il occupe la position 4,
- lorsque $R_7$ occupe la position 4, alors $R_8$ occupe la position 7,
- lorsque $R_7$ occupe la position 5, alors $R_8$ occupe la position 6.

Parmi les dérivés de benzimidazole de formule (II) ci-dessus, on peut plus particulièrement citer le 4-hydroxy benzimidazole, le 4-amino benzimidazole, le 4-hydroxy 7-méthyl benzimidazole, le 4-hydroxy 2-méthyl benzimidazole, le 1-butyl 4-hydroxy benzimidazole, le 4-amino 2-méthyl benzimidazole, le 5,6-dihydroxy benzimidazole, le 5-hydroxy 6-méthoxy benzimidazole, le 4,7-dihydroxy benzimidazole, le 4,7-dihydroxy 1-méthyl benzimidazole, le 4,7-diméthoxy benzimidazole, le 5,6-dihydroxy 1-méthyl benzimidazole, le 5,6-dihydroxy 2-méthyl benzimidazole, le 5,6-diméthoxy benzimidazole, et leurs sels d'addition avec un acide.

Parmi les dérivés de benzomorpholine utilisables à titre de coupleurs hétérocycliques dans la composition tinctoriale prête à l'emploi conforme à l'invention, on peut plus particulièrement citer les composés de formule (III) suivante, et leurs sels d'addition avec un acide :

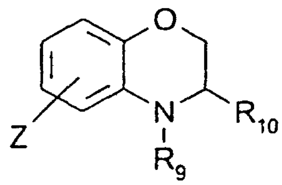

(III)

dans laquelle :

$R_9$ et $R_{10}$, identiques ou différents, représentent un atome d'hydrogène ou un radical alkyle en $C_1$-$C_4$,
Z représente un radical hydroxyle ou amino.

Parmi les dérivés de benzomorpholine de formule (III) ci-dessus, on peut plus particulièrement citer la 6-hydroxy 1,4-benzomorpholine, la N-méthyl 6-hydroxy 1,4-benzomorpholine, la 6-amino 1,4-benzomorpholine, et leurs sels d'addition avec un acide.

Parmi les dérivés de sésamol utilisables à titre de coupleur hétérocyclique dans la composition tinctoriale prête à l'emploi conforme à l'invention, on peut particulièrement citer les composés de formule (IV) suivante, et leurs sels d'addition avec un acide :

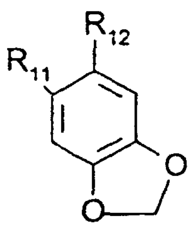

(IV)

dans laquelle :

$R_{11}$ désigne un radical hydroxyle, amino, alkyl($C_1$-$C_4$)amino, monohydroxyalkyl($C_1$-$C_4$)amino ou polyhydroxyalkyl ($C_2$-$C_4$)amino,
$R_{12}$ désigne un atome d'hydrogène ou d'halogène ou un radical alcoxy en $C_1$-$C_4$.

Parmi les dérivés de sésamol de formule (IV) ci-dessus, on peut plus particulièrement citer le 2-bromo 4,5-méthylènedioxy phénol, la 2-méthoxy 4,5-méthylènedioxy aniline, le 2-(β-hydroxyéthyl)amino 4,5-méthylènedioxy benzène, et leurs sels d'addition avec un acide.

Parmi les dérivés pyrazolo-azoliques utilisables à titre de coupleur hétérocyclique dans la composition tinctoriale prête à l'emploi conforme à l'invention, on peut plus particulièrement citer les composés décrits dans les demandes de brevets et brevets suivants : FR 2 075 583, EP-A-119 860, EP-A-285 274, EP-A-244 160, EP-A-578 248, GB 1 458 377, US 3 227 554, US 3 419 391, US 3 061 432, US 4 500 630, US 3 725 067, US 3 926 631, US 5 457 210, JP 84/99437, JP 83/42045, JP 84/162548, JP 84/171956, JP 85/33552, JP 85/43659, JP 85/172982, JP 85/190779 ainsi que dans les publications suivantes : Chem. Ber. 32, 797 (1899), Chem. Ber. 89, 2550, (1956), J. Chem. Soc. Perkin trans I, 2047, (1977), J. Prakt. Chem., 320, 533, (1978) ; dont les enseignements font partie intégrante de la présente demande.

A titre de dérivés pyrazolo-azoliques, on peut tout particulièrement citer :

- le 2-méthyl pyrazolo [1,5-b]-1,2,4-triazole,
- le 2-éthyl pyrazolo [1,5-b]-1,2,4-triazole,
- le 2-isopropyl pyrazolo [1,5-b]-1,2,4-triazole,
- le 2-phényl pyrazolo [1,5-b]-1,2,4-triazole,
- le 2,6-diméthyl pyrazolo [1,5-b]- 1,2,4-triazole ,
- le 7-chloro-2,6-diméthylpyrazolo[1,5-b]-1,2,4-triazole,
- le 3,6-diméthyl-pyrazolo [3,2-c]-1,2,4-triazole,
- le 6-phényl-3-méthylthio- pyrazolo [3,2-c]-1,2,4-triazole,
- le 6-amino- pyrazolo [1,5-a] benzimidazole,

et leurs sels d'addition avec un acide.

Parmi les dérivés pyrrolo-azoliques utilisables à titre de coupleur hétérocyclique dans la composition tinctoriale prête à l'emploi conforme à l'invention, on peut plus particulièrement citer les composés décrits dans les demandes de brevets et brevets suivants : US 5 256 526, EP-A-557 851, EP-A-578 248, EP-A-518 238, EP-A-456 226, EP-A-488 909, EP-A-488 248, et dans les publications suivantes :

- D.R. Liljegren Ber. 1964, 3436 ;
- E.J. Browne, J.C.S., 1962, 5149 ;
- P. Magnus, J.A.C.S., 1990, 112, 2465 ;
- P. Magnus, J.A.C.S., 1987, 109, 2711 ;
- Angew. Chem. 1960, 72, 956 ;
- et Rec. Trav. Chim. 1961, 80, 1075 ; dont les enseignements font partie intégrante de la présente demande.

A titre de dérivés pyrrolo-azoliques, on peut tout particulièrement citer :

- le 5-cyano-4-éthoxycarbonyl-8-méthyl pyrrolo [1,2-b]-1,2,4-triazole,
- le 5-cyano-8-méthyl-4-phényl pyrrolo [1,2-b]-1,2,4-triazole,
- le 7-amido-6-éthoxycarbonyl pyrrolo [1,2-a]- benzimidazole,

et leurs sels d'addition avec un acide.

Parmi les dérivés imidazolo-azoliques utilisables à titre de coupleur hétérocyclique dans la composition tinctoriale prête à l'emploi conforme à l'invention, on peut plus particulièrement citer les composés décrits dans les demandes de brevets et brevets suivants : US 5,441,863 ; JP 62-279 337 ; JP 06-236 011 et JP 07-092 632, dont les enseignements font partie intégrante de la présente demande.

A titre de dérivés imidazolo-azoliques, on peut tout particulièrement citer :

- le 7,8-dicyano-imidazolo- [3,2-a]- imidazole,
- le 7,8-dicyano-4-méthyl-imidazolo- [3,2-a]- imidazole,

et leurs sels d'addition avec un acide.

Parmi les dérivés pyrazolo-pyrimidiniques utilisables à titre de coupleur hétérocyclique dans la composition tinctoriale prête à l'emploi conforme à l'invention, on peut plus particulièrement citer les composés décrits dans la demande de brevet suivante : EP-A-304 001 dont l'enseignement fait partie intégrante de la présente demande.

A titre de dérivés pyrazolo-pyrimidiniques, on peut tout particulièrement citer :

- le pyrazolo [1,5-a] pyrimidin-7-one,
- le 2,5-diméthyl pyrazolo [1,5-a] pyrimidin-7-one,
- le 2-méthyl-6-éthoxycarbonyl pyrazolo [1,5-a] pyrimidin-7-one,
- le 2-méthyl-5-méthoxyméthyl pyrazolo [1,5-a] pyrimidin-7-one,
- le 2-ter-butyl-5-trifluorométhyl pyrazolo [1,5-a] pyrimidin-7-one,
- 2,7-diméthyl pyrazolo [1,5-a] pyrimidin-5-one,

et leurs sels d'addition avec un acide.

Parmi les dérivés de pyrazolin-3,5-diones utilisables à titre de coupleur hétérocyclique dans la composition tinctoriale prête à l'emploi conforme à l'invention, on peut plus particulièrement citer les composés décrits dans les demandes de brevets et brevets suivants : JP 07-036159, JP 07-084348 et US 4 128 425, et dans les publications suivantes :

- L. WYZGOWSKA, Acta. Pol. Pharm. 1982, 39 (1-3), 83.
- E. HANNIG, Pharmazie, 1980, 35 (4), 231
- M. H. ELNAGDI, Bull. Chem. Soc. Jap., 46 (6), 1830, 1973
- G. CARDILLO, Gazz. Chim. Ital. 1966, 96, (8-9), 973.

dont les enseignements font partie intégrante de la présente demande.

A titre de dérivés de pyrazolin-3,5-diones, on peut tout particulièrement citer :

- la 1,2-diphényl pyrazolin-3,5-dione,
- la 1,2-diéthyl pyrazolin-3,5-dione,

et leurs sels d'addition avec un acide.

Parmi les dérivés pyrrolo-[3,2-d]-oxazoliques utilisables à titre de coupleur hétérocyclique dans la composition tinctoriale prête à l'emploi conforme à l'invention, on peut plus particulièrement citer les composés décrits dans la demande de brevet JP 07 325 375 dont l'enseignement fait partie intégrante de la présente demande.

Parmi les dérivés pyrazolo-[3,4-d]-thiazoliques utilisables à titre de coupleur hétérocyclique dans la composition tinctoriale prête à l'emploi conforme à l'invention, on peut plus particulièrement citer les composés décrits dans la demandes de brevet JP 07 244 361 et dans J. Heterocycl. Chem. 16, 13, (1979).

Parmi les dérivés S-oxyde-thiazolo-azoliques et S,S-dioxyde-thiazolo-azoliques utilisables à titre de coupleur hétérocyclique dans la composition tinctoriale prête à l'emploi conforme à l'invention, on peut plus particulièrement citer les composés décrits dans les documents suivants :

- JP 07 098489 ;
- Khim. Geterotsilk. Soedin, 1967, p. 93 ;
- J. Prakt. Chem., 318, 1976, p. 12 ;
- Indian J. Heterocycl. Chem. 1995, 5 (2), p. 135 ;
- Acta. Pol. Pharm. 1995, 52 (5), 415 ;
- Heterocycl. Commun. 1995, 1 (4), 297 ;
- Arch. Pharm. (Weinheim, Ger.), 1994, 327 (12), 825.

Lorsqu'ils sont présents ce ou ces coupleurs hétérocycliques représentent de préférence de 0,0001 à 10 % en poids environ du poids total de la composition tinctoriale prête à l'emploi et encore plus préférentiellement de 0,005 à 5 % en poids environ de ce poids.

La composition tinctoriale prête à l'emploi conforme à l'invention peut encore contenir, en plus des colorants définis ci-dessus, d'autres bases d'oxydation et/ou d'autres coupleurs et/ou d'autres colorants comme par exemple des colorants directs, notamment pour modifier les nuances ou les enrichir en reflets.

Parmi les bases d'oxydation pouvant être présentes à titre additionnel dans la composition tinctoriale prête à l'emploi conforme à l'invention, on peut notamment citer les paraphénylènediamines, les bis-phènylalkylènediamines, les orthophénylènediamines, les para-aminophénols, les orthoaminophénols, et leurs sels d'addition avec un acide.

Lorsqu'elles sont utilisées, ces bases d'oxydation additionnelles représentent de préférence de 0,0005 à 12 % en poids environ du poids total de la composition tinctoriale, et encore plus préférentiellement de 0,005 à 6 % en poids environ de ce poids.

Parmi les coupleurs pouvant être présents à titre additionnel dans la composition tinctoriale conforme prête à l'emploi conforme à l'invention, on peut notamment citer les méta-phénylènediamines, les méta-aminophénols, les métadiphénols, et leurs sels d'addition avec un acide.

Lorsqu'ils sont présents ces coupleurs additionnels représentent de préférence de 0,0001 à 10 % en poids environ du poids total de la composition tinctoriale prête à l'emploi et encore plus préférentiellement de 0,005 à 5 % en poids environ de ce poids.

D'une manière générale, les sels d'addition avec un acide utilisables dans le cadre des compositions tinctoriales de l'invention (bases d'oxydation et coupleurs) sont notamment choisis parmi les chlorhydrates, les bromhydrates, les sulfates et les tartrates, les lactates et les acétates.

Le milieu approprié pour la teinture (ou support) de la composition tinctoriale prête à l'emploi conforme à l'invention est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols en $C_1$-$C_4$, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition prête à l'emploi conforme à l'invention est choisi de telle manière que l'activité enzymatique de l'oxydo-réductase à 2 électrons ne soit pas altérée. Il est généralement compris entre 5 et 11 environ, et de préférence entre 6,5 et 10 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines, le 2-méthyl 2-amino propanol ainsi que leurs dérivés, les hydroxydes de sodium ou de potassium et les composés de formule (V) suivante :

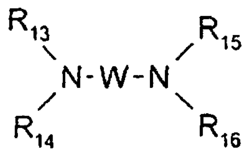

(V)

dans laquelle W est un reste propylène éventuellement substitué par un groupement hydroxyle ou un radical alkyle en $C_1$-$C_4$ ; $R_{13}$, $R_{14}$, $R_{15}$ et $R_{16}$, identiques ou différents, représentent un atome d'hydrogène, un radical alkyle en $C_1$-$C_4$ ou hydroxyalkyle en $C_1$-$C_4$.

La composition tinctoriale prête à l'emploi conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des enzymes différentes des oxydo-réductases à 2 électrons utilisées conformément à l'invention telles que par exemples des peroxydases, des agents de pénétration, des agents séquestrants, des parfums, des tampons, des agents dispersants, des agents de conditionnement tels que par exemple des silicones, des agents filmogènes, des agents conservateurs, des agents opacifiants.

Bien entendu, l'homme de l'art veillera à choisir ce ou ces éventuels composés complémentaires de manière telle que les propriétés avantageuses attachées intrinsèquement à la composition tinctoriale prête à l'emploi conforme à l'invention ne soient pas, ou substantiellement pas, altérées par la ou les adjonctions envisagées.

La composition tinctoriale prête à l'emploi conforme à l'invention peut se présenter sous des formes diverses, telles que sous forme de liquides, de crèmes, de gels, éventuellement pressurisés, ou sous toute autre forme appropriée pour réaliser une teinture des fibres kératiniques, et notamment des cheveux humains. Dans ce cas, le ou les colorants d'oxydation et la ou les oxydo-réductases à 2 électrons sont présents au sein de la même composition prête à l'emploi, et par conséquent ladite composition doit être exempte d'oxygène gazeux, de manière à éviter toute oxydation prématurée du ou des colorants d'oxydation.

L'invention a également pour objet un procédé de teinture des fibres kératiniques et en particulier des fibres kératiniques humaines telles que les cheveux mettant en oeuvre la composition tinctoriale prête à l'emploi telle que définie précédemment.

Selon ce procédé, on applique sur les fibres au moins une composition tinctoriale prête à l'emploi telle que définie précédemment, pendant un temps suffisant pour développer la coloration désirée, après quoi on rince, on lave éventuellement au shampooing, on rince à nouveau et on sèche.

Le temps nécessaire au développement de la coloration sur les fibres kératiniques est généralement compris entre 3 et 60 minutes et encore plus précisément 5 et 40 minutes.

Selon une forme de réalisation particulière de l'invention, le procédé comporte une étape préliminaire consistant à stocker sous forme séparée, d'une part, une composition (A) comprenant, dans un milieu approprié pour la teinture, au moins une base d'oxydation hétérocyclique telle que définie précédemment et/ou au moins un coupleur hétérocyclique tel que défini précédemment et, d'autre part, une composition (B) renfermant, dans un milieu approprié pour la teinture, au moins une enzyme de type oxydo-réductase à 2 électrons en présence d'au moins un donneur pour ladite enzyme, puis à procéder à leur mélange au moment de l'emploi avant d'appliquer ce mélange sur les fibres kératiniques.

Les compositions A et B conformes à l'invention peuvent être conditionnées dans un dispositif à plusieurs

compartiments ou "kit" de teinture ou tout autre système de conditionnement à plusieurs compartiments dont un premier compartiment renferme la composition (A) telle que définie ci-dessus et un second compartiment renferme la composition (B) telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

## EXEMPLES

## EXEMPLES 1 ET 2 COMPARATIFS

On a préparé les compositions tinctoriales prêtes à l'emploi suivantes (teneurs en grammes) :

| COMPOSITION | 1 (*) | 2 |
|---|---|---|
| Paraphénylènediamine (base d'oxydation benzénique) | 0,324 | - |
| Sulfate de 2,4,5,6-tétra-aminopyrimidine (base d'oxydation hétérocyclique) | - | 0,714 |
| Résorcine (coupleur benzénique) | 0,33 | 0,33 |
| Uricase d'Arthrobacter globiformis à 20 Unités Internationales (U.I.) / mg, commercialisée par la société Sigma | 1,5 | 1,5 |
| Acide urique | 1,5 | 1,5 |
| Support de teinture commun (**) | (**) | (**) |
| Eau déminéralisée q.s.p. | 100 g | 100 g |

(*) Exemple ne faisant pas partie de l'invention

(**) : Support de teinture commun :
- Ethanol 10,0 g
- Gomme de guar hydroxypropylée vendue sous la dénomination JAGUAR HP 60 par la société MAYHALL 0,8 g
- Alkyl ($C_8$-$C_{10}$) polyglucoside en solution aqueuse à 60 % de matière active (M.A.) tamponné par du citrate d'ammonium (0,5%), vendu sous la dénomination ORAMIX CG110 par la société SEPPIC 8,0 g
- Monoéthanolamine q.s. pH = 9,5

Il est important de noter que chacune des compositions tinctoriales prêtes à l'emploi décrites ci-dessus contient la même quantité molaire de chacune des bases d'oxydation, à savoir $3.10^{-3}$ mole.

Chacune des compositions tinctoriales prêtes à l'emploi décrites ci-dessus a été appliquée sur des mèches de cheveux gris naturels à 90 % de blancs pendant 30 minutes. Les cheveux ont ensuite été rincés, lavés avec un shampooing standard, puis séchés.

La couleur des mèches a été ensuite évaluée avant et après la coloration dans le système MUNSELL au moyen d'un colorimètre CM 2002 MINOLTA de façon à déterminer la puissance des colorations obtenues avec chacune des compositions décrites ci-dessus.

La différence entre la couleur de la mèche avant la teinture et la couleur de la mèche après la teinture a été calculée en appliquant la formule de NICKERSON :

$$\Delta E = 0{,}4\ Co\Delta H + 6\Delta V + 3\ \Delta C$$

telle que décrite par exemple dans "Couleur, Industrie et Technique" ; pages 14-17 ; vol. n° 5 ; 1978.

Dans cette formule, $\Delta E$ représente la différence de couleur entre deux mèches, $\Delta H$, $\Delta V$ et $\Delta C$ représentent la variation en valeur absolue des paramètres H, V et C et Co représente la pureté de la mèche par rapport à laquelle on désire évaluer la différence de couleur.

La puissance de la coloration ($\Delta E$) est d'autant plus importante que le chiffre indiqué est élevé.

Les résultats sont donnés dans le tableau I ci-dessous :

Tableau I

| EXEMPLE | Couleur des cheveux avant la teinture | Couleur des cheveux après la teinture | Puissance de la coloration | | | |
|---|---|---|---|---|---|---|
| | | | ΔH | ΔV | ΔC | **ΔE** |
| **1 (*)** | 3,2 Y 5,0 / 1,6 | 3,2 Y 3,6 / 2,2 | 0 | 1,4 | 0,6 | **10,2** |
| **2** | 3,2 Y 5,0 / 1,6 | 8,3 R 3,7 / 2,9 | 14,9 | 1,3 | 1,3 | **21,2** |

(*) : Exemple ne faisant pas partie de l'invention.

Ces résultats montrent qu'en présence du système oxydant Uricase/Acide urique, la composition tinctoriale prête à l'emploi de l'exemple 1 ne faisant pas partie de l'invention car ne contenant pas de colorant d'oxydation hétérocyclique conduit à une coloration nettement moins puissante que la composition tinctoriale prête à l'emploi de l'exemple 2 conforme à l'invention et qui contient au moins un composé hétérocyclique, à savoir une base d'oxydation hétérocyclique qui est la 2,4,5,6-tétra-aminopyrimidine.

**EXEMPLES 3 ET 4 COMPARATIFS**

On a préparé les compositions tinctoriales prêtes à l'emploi suivantes (teneurs en grammes) :

| **COMPOSITION** | **3 (*)** | **4 (*)** |
|---|---|---|
| Paraphénylènediamine (base d'oxydation benzénique) | 0,648 | - |
| Sulfate de 2,4,5,6-tétra-aminopyrimidine (base d'oxydation hétérocyclique) | - | 1,428 |
| Résorcine (coupleur benzénique) | 0,66 | 0,66 |
| Support de teinture commun (***) | (***) | (***) |
| Eau déminéralisée q.s.p. | 100 g | 100 g |

(*) Exemple ne faisant pas partie de l'invention

(***) Support de teinture commun :
- Ethanol 20,0 g
- Gomme de guar hydroxypropylée vendue sous la dénomination JAGUAR HP 60 par la société MAYHALL 1,6 g
- Alkyl ($C_8$-$C_{10}$) polyglucoside en solution aqueuse à 60 % de matière active (M.A.) tamponné par du citrate d'ammonium (0,5%), vendu sous la dénomination ORAMIX CG110 par la société SEPPIC 8,0 g
- Monoéthanolamine q.s. pH = 9,5

Il est important de noter que chacune des compositions tinctoriales prêtes à l'emploi décrites ci-dessus contient la même quantité molaire de chacune des bases d'oxydation, à savoir $6.10^{-3}$ mole.

Au moment de l'emploi, chacune des compositions tinctoriales prêtes à l'emploi décrites ci-dessus a été mélangée avec une quantité égale en poids d'une solution de peroxyde d'hydrogène à 20 volumes (6% en poids).

Chacun des mélanges résultant a été appliqué sur des mèches de cheveux gris naturels à 90 % de blancs pendant 30 minutes. Les mèches de cheveux ont ensuite été rincées, lavées avec un shampooing standard, rincées à nouveau puis séchés.

Comme précédemment décrit pour les exemples 1 et 2 ci-dessus, la coloration a été évaluée dans le système MUNSELL, avant et après la teinture, au moyen d'un colorimètre CM2002 MINOLTA.

La puissance de la coloration a été calculée en appliquant la formule de NICKERSON.

Les résultats sont rassemblés dans le tableau II ci-dessous :

Tableau II

| EXEMPLE | Couleur des cheveux avant la teinture | Couleur des cheveux après la teinture | Puissance de la coloration | | | |
|---|---|---|---|---|---|---|
| | | | ΔH | ΔV | ΔC | **ΔE** |
| **3(*)** | 2,9 Y 5,9 / 1,5 | 9,3 YR 2,5 / 1,2 | 3,6 | 3,4 | 0,3 | **23,5** |
| **4 (*)** | 2,9 Y 5,9 / 1,5 | 2,5 YR 4,4 / 3,0 | 10,4 | 1,5 | 1,5 | **19,7** |

(*) : Exemple ne faisant pas partie de l'invention.

Contrairement à ce qui a été démontré précédemment pour les exemples comparatifs 1 et 2, ces résultats montrent qu'en employant un système oxydant classique ne faisant pas partie de l'invention, comme par exemple ici du peroxyde d'hydrogène, le fait de remplacer un colorant benzénique par un colorant hétérocyclique (ici la paraphénylènediamine a été remplacée par la 2,4,5,6-tétra-aminopyrimidine), ne permet pas d'augmenter la puissance des colorations obtenues.

## EXEMPLES 5 ET 6 COMPARATIFS

On a préparé les compositions tinctoriales prêtes à l'emploi suivantes (teneurs en grammes) :

| COMPOSITION | 5 (*) | 6 |
|---|---|---|
| Dichlorhydrate de 2,5-diaminopyridine (base d'oxydation exclue de l'invention) | 0,546 | - |
| Sulfate de 2,4,5,6-tétra-aminopyrimidine (base d'oxydation hétérocyclique) | - | 0,714 |
| Résorcine (coupleur benzénique) | 0,33 | 0,33 |
| Uricase d'Arthrobacter globiformis à 20 U.I / mg, commercialisée par la société Sigma | 1,0 | 1,0 |
| Acide urique | 1,0 | 1,0 |
| Support de teinture commun (**) | (**) | (**) |
| Eau déminéralisée q.s.p. | 100 g | 100 g |

(*) Exemple ne faisant pas partie de l'invention

(**) : Support de teinture commun :
il est identique à celui utilisé pour les exemples 1 et 2 ci-dessus.

Il est important de noter que chacune des compositions tinctoriales prêtes à l'emploi décrites ci-dessus contient la même quantité molaire de chacune des bases d'oxydation, à savoir $3.10^{-3}$ mole.

Chacune des compositions tinctoriales prêtes à l'emploi décrites ci-dessus a été appliquée sur des mèches de cheveux gris naturels à 90 % de blancs pendant 30 minutes. Les cheveux ont ensuite été rincés, lavés avec un shampooing standard, puis séchés.

Comme précédemment décrit pour les exemples 1 et 2 ci-dessus, la coloration a été évaluée dans le système MUNSELL, avant et après la teinture, au moyen d'un colorimètre CM2002 MINOLTA.

La puissance de la coloration a été calculée en appliquant la formule de NICKERSON.

Les résultats sont rassemblés dans le tableau III ci-dessous :

Tableau III

| EXEMPLE | Couleur des cheveux avant la teinture | Couleur des cheveux après la teinture | Puissance de la coloration | | | |
|---|---|---|---|---|---|---|
| | | | ΔH | ΔV | ΔC | **ΔE** |
| **5 (*)** | 2,9 Y 5,9 / 1,5 | 4,8 YR 4,8 / 2,1 | 8,1 | 1,1 | 0,6 | **13,3** |
| **6** | 2,9 Y 5,9 / 1,5 | 10,0 R 4,7 / 2,6 | 12,9 | 1,2 | 1,1 | **18,2** |

(*) : Exemple ne faisant pas partie de l'invention.

Ces résultats montrent qu'en présence du système oxydant Uricase/Acide urique, la composition tinctoriale prête à l'emploi de l'exemple 5 ne faisant pas partie de l'invention car contenant de la 2,5-diaminopyridine qui est une base d'oxydation pyridinique monocyclique exclue de l'invention conduit à une coloration nettement moins puissante que la composition tinctoriale prête à l'emploi de l'exemple 6 conforme à l'invention et qui contient au moins un composé hétérocyclique conforme à l'invention, à savoir une base d'oxydation hétérocyclique qui est la 2,4,5,6-tétra-aminopyrimidine.

## EXEMPLES 7 A 13 DE TEINTURE

On a préparé les compositions tinctoriales prêtes à l'emploi conformes à l'invention, suivantes (teneurs en grammes) :

| EXEMPLE | 7 | 8 | 9 | 10 | 11 | 12 | 13 |
|---|---|---|---|---|---|---|---|
| Pyrazolo-[1,5-a]-pyrimidine-3,7-diamine, 2 HCl | 0,666 | 0,666 | - | - | - | - | - |
| 3-amino-7-β-hydroxyéthylamino-5-méthyl-pyrazolo-[1,5-a]-pyrimidine, HCl | - | - | 0,729 | - | - | - | - |
| Paraphénylènediamine | - | - | - | 0,216 | 0,216 | - | - |
| 4,5-diamino 1-éthyl 3-méthyl pyrazole, 2HCl | - | - | - | - | - | 0,639 | 0,639 |
| 2-méthyl 5-amino phénol | 0,369 | - | - | - | - | - | - |
| 6-hydroxy 1,4-benzomorpholine | - | 0,453 | - | - | - | - | - |
| 1-méthyl 2-hydroxy 4-(β-hydroxyéthyl)amino benzène | - | - | 0,501 | - | - | - | - |
| 2-méthoxy 4,5-méthylènedioxy aniline, HCl | - | - | - | 0,407 | - | - | - |
| 2,6-diméthyl-pyrazolo [1,5-b]-1,2,4-triazole, paratoluènesulfonate | - | - | - | - | 0,617 | - | - |
| Méta-aminophénol | - | - | - | - | - | 0,327 | - |
| 1-β-hydroxyéthyloxy 2,4-diaminobenzène, 2HCl | - | - | - | - | - | - | 0,723 |
| Uricase d'Arthrobacter globiformis à 20 U.I / mg, commercialisée par la société Sigma | 0,8 | 1,2 | 1,0 | 0,8 | 1,0 | 2,0 | 1,8 |
| Acide urique | 1,2 | 1,5 | 2,0 | 0,8 | 1,5 | 2,0 | 1,5 |
| Support de teinture commun (**) | (**) | (**) | (**) | (**) | (**) | (**) | (**) |
| Eau déminéralisée q.s.p. | 100 g | 100 g | 100 g | 100 g | 100 g | 100 g | 100 g |

(**) : Support de teinture commun :

Il est identique à celui utilisé pour les exemples 1 et 2 ci-dessus.

Chacune des compositions tinctoriales prêtes à l'emploi décrites ci-dessus a été appliquée sur des mèches de cheveux gris à 90 % de blancs, naturels ou permanentés, pendant 30 minutes. Les cheveux ont ensuite été rincés, lavés avec un shampooing standard, puis séchés.

Les mèches de cheveux ont été teintes dans les nuances figurant dans le tableau IV ci-dessous.

Tableau IV

| EXEMPLE | Nuance obtenue sur cheveux naturels | Nuance obtenue sur cheveux permanentés |
|---|---|---|
| **7** | Irisé doré | Irisé doré |
| **8** | Irisé | Irisé |
| **9** | Irisé cuivré | Irisé cuivré |
| **10** | Cendré doré | Cendré mat |
| **11** | Irisé intense | Irisé intense |
| **12** | Violine | Violine |
| **13** | Violet | Violet |

**Revendications**

1. Composition prête à l'emploi, pour la teinture d'oxydation des fibres kératiniques et en particulier des fibres kératiniques humaines telles que les cheveux, comprenant l'association d'au moins une base d'oxydation et d'au moins un coupleur, **caractérisée par le fait qu'**elle comprend, dans un milieu approprié pour la teinture, au moins une enzyme de type oxydo-réductase à 2 électrons en présence d'au moins un donneur pour ladite enzyme, et que ladite base d'oxydation et/ou ledit coupleur est choisi parmi les composés hétérocycliques à l'exclusion des composés pyridiniques monocycliques.

2. Composition selon la revendication 1, **caractérisée par le fait que** l'oxydo-réductases à 2 électrons est choisie parmi les uricases d'origine animale, microbiologique ou biotechnologique.

3. Composition selon la revendication 1 ou 2, **caractérisée par le fait que** la ou les oxydo-réductases à 2 électrons représentent de 0,01 à 20 % en poids du poids total de la composition tinctoriale prête à l'emploi.

4. Composition selon la revendication 3, **caractérisée par le fait que** la ou les oxydo-réductases à 2 électrons représentent de 0,1 à 5 % en poids du poids total de la composition tinctoriale prête à l'emploi.

5. Composition selon la revendication 2, **caractérisée par le fait que** le donneur (ou substrat) pour ladite oxydo-réductase à 2 est choisi parmi l'acide urique et ses sels.

6. Composition selon l'une quelconque des revendications précédentes, **caractérisée par le fait que** le ou les donneurs représentent de 0,01 à 20 % en poids du poids total de la composition tinctoriale prête à l'emploi.

7. Composition selon la revendication 6, **caractérisée par le fait que** le ou les donneurs représentent de 0,1 à 5 % en poids du poids total de la composition tinctoriale prête à l'emploi.

8. Composition selon l'une quelconque des revendications précédentes,
**caractérisée par le fait que** la ou les bases d'oxydation hétérocycliques sont choisies parmi les dérivés pyrimidiniques, les dérivés pyrazoliques, et leurs sels d'addition avec un acide.

**9.** Composition selon la revendication 8, **caractérisée par le fait que** les dérivés pyrimidiniques sont choisis parmi la 2,4,5,6-tétra-aminopyrimidine, la 4-hydroxy 2,5,6-triaminopyrimidine, les dérivés pyrazolopyrimidiniques, et leurs sels d'addition avec un acide.

**10.** Composition selon la revendication 9, **caractérisée par le fait que** les dérivés pyrazolopyrimidiniques sont choisis parmi la pyrazolo-[1,5-a]-pyrimidine-3,7-diamine, la 2-méthyl pyrazolo-[1,5-a]-pyrimidine-3,7-diamine, la 2,5-diméthyl pyrazolo-[1,5-a]-pyrimidine-3,7-diamine, la pyrazolo-[1,5-a]-pyrimidine-3,5-diamine, la 2,7-diméthyl pyrazolo-[1,5-a]-pyrimidine-3,5-diamine, le 3-amino pyrazolo-[1,5-a]-pyrimidin-7-ol, le 3-amino 5-méthyl pyrazolo-[1,5-a]-pyrimidin-7-ol, le 3-amino pyrazolo-[1,5-a]-pyrimidin-5-ol, le 2-(3-amino pyrazolo-[1,5-a]-pyrimidin-7-ylamino)-éthanol, la 3-amino-7-β-hydroxyéthylamino-5-méthyl-pyrazolo-[1,5-a]-pyrimidine, le 2-(7-amino pyrazolo-[1,5-a]-pyrimidin-3-ylamino)-éthanol, le 2-[(3-amino-pyrazolo-[1,5-a]-pyrimidin-7-yl)-(2-hydroxyéthyl)-amino]-éthanol, le 2-[(7-amino-pyrazolo-[1,5-a]-pyrimidin-3-yl)-(2-hydroxyéthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N-7, N-7-tetraméthyl pyrazolo-[1,5-a]-pyrimidine-3,7-diamine, et leurs sels d'addition et leurs formes tautomères, lorsqu'il existe un équilibre tautomérique.

**11.** Composition selon la revendication 8, **caractérisée par le fait que** les dérivés pyrazoliques sont choisis parmi le 4,5-diamino pyrazole, le 4,5-diamino 1-méthyl pyrazole, le 1-benzyl 4,5-diamino pyrazole, le 3,4-diamino pyrazole, le 1-benzyl 4,5-diamino 3-méthyl pyrazole, le 4-amino 1,3-diméthyl 5-hydrazino pyrazole, le 4,5-diamino 3-méthyl 1-phényl pyrazole, le 4,5-diamino 3-méthyl 1-tert-butyl pyrazole, le 4,5-diamino 1-méthyl 3-tert-butyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, et leurs sels d'addition avec un acide.

**12.** Composition selon l'une quelconque des revendications précédentes, **caractérisée par le fait que** la ou les bases d'oxydation hétérocycliques représentent de 0,0005 à 12 % en poids du poids total de la composition tinctoriale prête à l'emploi.

**13.** Composition selon la revendication 12, **caractérisée par le fait que** la ou les bases d'oxydation hétérocycliques représentent de 0,005 à 6 % en poids du poids total de la composition tinctoriale prête à l'emploi.

**14.** Composition selon l'une quelconque des revendications précédentes, **caractérisée par le fait que** le ou les coupleurs hétérocycliques sont choisis parmi les dérivés indoliques, les dérivés indoliniques, les dérivés de benzimidazole, les dérivés de benzomorpholine, les dérivés de sésamol, les dérivés pyrazolo-azoliques, les dérivés pyrrolo-azoliques, les dérivés imidazolo-azoliques, les dérivés pyrazolo-pyrimidiniques, les dérivés de pyrazolin-3,5-diones, les dérivés pyrrolo-[3,2-d]-oxazoliques, les dérivés pyrazolo-[3,4-d]-thiazoliques, les dérivés S-oxyde-thiazolo-azoliques, les dérivés S,S-dioxyde-thiazolo-azoliques, et leurs sels d'addition avec un acide.

**15.** Composition selon la revendication 14, **caractérisée par le fait que** les dérivés indoliques sont choisis parmi les composés de formule (I) suivante, et leurs sels d'addition avec un acide :

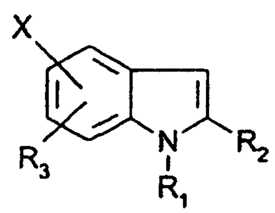

(I)

dans laquelle :

$R_1$ représente un atome d'hydrogène, un radical alkyle en $C_1$-$C_4$, mon- ou polyhydroxyalkyle en $C_2$-$C_4$ ou aminoalkyle en $C_1$-$C_4$ dont l'amine est mono ou disubstituée par un groupement alkyle en $C_1$-$C_4$ ;
$R_2$ représenté un atome d'hydrogène ou un radical alkyle en $C_1$-$C_4$ ;
$R_3$ représente un atome d'hydrogène, un radical alkyle en $C_1$-$C_4$ ou hydroxyle ;
X représente un radical hydroxyle ou $NHR_4$ dans lequel $R_4$ représente un atome d'hydrogène, un radical alkyle en $C_1$-$C_4$ ou hydroxyalkyle en $C_1$-$C_4$.

**16.** Composition selon la revendication 15, **caractérisée par le fait que** les dérivés indoliques sont choisis parmi le 4-hydroxy indole, le 6-hydroxy indole, le 7-amino indole, le 6-amino indole, le 7-hydroxy indole, le 7-éthyl 6-(β-hydroxyéthyl)amino indole, le 4-amino indole, le 6-hydroxy 1-méthyl indole, le 5,6-dihydroxy indole, le 4-hydroxy 1-N-méthyl indole, le 4-hydroxy 2-méthyl indole, le 4-hydroxy 5-méthyl indole, le 4-hydroxy 1-N-(β-hydroxyéthyl) indole, le 4-hydroxy 1-N-(β-hydroxypropyl) indole, le 1-N-(β,γ-dihydroxypropyl) 4-hydroxy indole, le 4-hydroxy 1-N-(β-hydroxyéthyl) 5-méthyl indole, le 1-N-(γ-diméthylaminopropyl) 4-hydroxy indole, et leur sels d'addition avec un acide.

**17.** Composition selon la revendication 14, **caractérisée par le fait que** les dérivés indoliniques sont choisis parmi la 4-hydroxy indoline, la 6-hydroxy indoline, la 6-amino indoline, la 5,6-dihydroxy indoline, et leurs sels d'addition avec un acide.

**18.** Composition selon la revendication 14, **caractérisée par le fait que** les dérivés de benzimidazole sont choisis parmi les composés de formule (II) suivante, et leurs sels d'addition avec un acide :

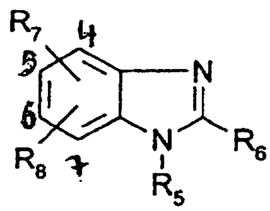

(II)

dans laquelle :

$R_5$ représente un atome d'hydrogène ou un radical alkyle en $C_1$-$C_4$,
$R_6$ représente un atome d'hydrogène, un radical alkyle en $C_1$-$C_4$ ou phényle,
$R_7$ représente un radical hydroxyle, amino ou méthoxy,
$R_8$ représente un atome d'hydrogène, un radical hydroxyle, méthoxy ou alkyle en $C_1$-$C_4$ ;

sous réserve que :

- lorsque $R_7$ désigne un radical amino, alors il occupe la position 4,
- lorsque $R_7$ occupe la position 4, alors $R_8$ occupe la position 7,
- lorsque $R_7$ occupe la position 5, alors $R_8$ occupe la position 6.

**19.** Composition selon la revendication 18, **caractérisée par le fait que** les dérivés de benzimidazole sont choisis le 4-hydroxy benzimidazole, le 4-amino benzimidazole, le 4-hydroxy 7-méthyl benzimidazole, le 4-hydroxy 2-méthyl benzimidazole, le 1-butyl 4-hydroxy benzimidazole, le 4-amino 2-méthyl benzimidazole, le 5,6-dihydroxy benzimidazole, le 5-hydroxy 6-méthoxy benzimidazole, le 4,7-dihydroxy benzimidazole, le 4,7-dihydroxy 1-méthyl benzimidazole, le 4,7-diméthoxy benzimidazole, le 5,6-dihydroxy 1-méthyl benzimidazole, le 5,6-dihydroxy 2-méthyl benzimidazole, le 5,6-diméthoxy benzimidazole, et leurs sets d'addition avec un acide.

**20.** Composition selon la revendication 14, **caractérisée par le fait que** les dérivés de benzomorpholine sont choisis parmi les composés de formule (III) suivante, et leurs sels d'addition avec un acide :

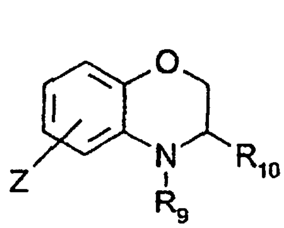

(III)

dans laquelle :

$R_9$ et $R_{10}$, identiques ou différents, représentent un atome d'hydrogène ou un radical alkyle en $C_1$-$C_4$,

Z représente un radical hydroxyle ou amino.

**21.** Composition selon la revendication 20, **caractérisée par le fait que** les dérivés de benzomorpholine sont choisis parmi la 6-hydroxy 1,4-benzomorpholine, la N-méthyl 6-hydroxy 1,4-benzomorpholine, la 6-amino 1,4-benzomorpholine, et leurs sels d'addition avec un acide.

**22.** Composition selon la revendication 14, **caractérisée par le fait que** les dérivés de sésamol sont choisis parmi les composés de formule (IV) suivante, et leurs sels d'addition avec un acide :

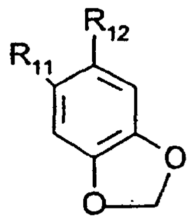

(IV)

dans laquelle :

$R_{11}$ désigne un radical hydroxyle, amino, alkyl($C_1$-$C_4$)amino, monohydroxyalkyl($C_1$-$C_4$)amino ou polyhydroxyalkyl($C_2$-$C_4$)amino,
$R_{12}$ désigne un atome d'hydrogène ou d'halogène ou un radical alcoxy en $C_1$-$C_4$.

**23.** Composition selon la revendication 22, **caractérisée par le fait que** les dérivés de sésamol sont choisis parmi le 2-bromo 4,5-méthylènedioxy phénol, la 2-méthoxy 4,5-méthylènedioxy aniline, le 2-(β-hydroxyéthyl)amino 4,5-méthylènedioxy benzène, et leurs sels d'addition avec un acide.

**24.** Composition selon la revendication 14, **caractérisée par le fait que** les dérivés pyrazolo-azoliques sont choisis parmi :

- le 2-méthyl pyrazolo [1,5-b]-1,2,4-triazole,
- le 2-éthyl pyrazolo [1,5-b]-1,2,4-triazole,
- le 2-isopropyl pyrazolo [1,5-b]-1,2,4-triazole,
- le 2-phényl pyrazolo [1,5-b]-1,2,4-triazole,
- le 2,6-diméthyl pyrazolo [1,5-b]-1,2,4-triazole ,
- le 7-chloro-2,6-diméthylpyrazolo[1,5-b]-1,2,4-triazole,
- le 3,6-diméthyl-pyrazolo [3,2-c]-1,2,4-triazole,
- le 6-phényl-3-méthylthio- pyrazolo [3,2-c]-1,2,4-triazole,
- le 6-amino- pyrazolo [1,5-a] benzimidazole,

et leurs sels d'addition avec un acide.

**25.** Composition selon, la revendication 14, **caractérisée par le fait que** les dérivés pyrrolo-azoliques sont choisis parmi :

- le 5-cyano-4-éthoxycarbonyl-8-méthyl pyrrolo [1,2-b]-1,2,4-triazole,
- le 5-cyano-8-méthyl-4-phényl pyrrolo [1,2-b]-1,2,4-triazole,
- le 7-amido-6-éthoxycarbonyl pyrrolo [1,2-a]- benzimidazole,

et leurs sels d'addition avec un acide.

**26.** Composition selon la revendication 14, **caractérisée par le fait que** les dérivés imidazolo-azoliques sont choisis parmi :

- le 7,8-dicyano-imidazolo- [3,2-a]- imidazole,
- le 7,8-dicyano-4-méthyl-imidazolo- [3,2-a]- imidazole,

et leurs sels d'addition avec un acide.

**27.** Composition selon la revendication 14, **caractérisée par le fait que** les dérivés pyrazolo-pyrimidiniques sont choisis parmi :

- le pyrazolo [1,5-a] pyrimidin-7-one,
- le 2,5-diméthyl pyrazolo [1,5-a] pyrimidin-7-one,
- le 2-méthyl-6-éthoxycarbonyl pyrazolo [1,5-a] pyrimidin-7-one,
- le 2-méthyl-5-méthoxyméthyl pyrazolo [1,5-a] pyrimidin-7-one,
- le 2-ter-butyl-5-trifluorométhyl pyrazolo [1,5a] pyrimidin-7-one,
- 2,7-diméthyl pyrazolo [1,5-a] pyrimidin-5-one,

et leurs sels d'addition avec un acide.

**28.** Composition selon la revendication 14, **caractérisée par le fait que** les dérivés de pyrazolin-3,5-diones sont choisis parmi :

- la 1,2-diphényl pyrazolin-3,5-dione,
- la 1,2-diéthyl pyrazolin-3,5-dione,

et leurs sels d'addition avec un acide.

**29.** Composition selon l'une quelconque des revendications précédentes, **caractérisée par le fait que** le ou les coupleurs hétérocycliques représentent de 0,0001 à 10 % en poids du poids total de la composition tinctoriale prête à l'emploi.

**30.** Composition selon la revendication 29, **caractérisée par le fait que** le ou les coupleurs hétérocycliques représentent de 0,005 à 5 % en poids du poids total de la composition tinctoriale prête à l'emploi.

**31.** Composition selon l'une quelconque des revendications précédentes, **caractérisée par le fait qu'**elle contient au moins une base d'oxydation additionnelle choisie parmi les paraphénylénediamines, les bis-phénylalkylènediamines, les orthophénylènediamines, les para-aminophénols, les orthoaminophénols, et leurs sels d'addition avec un acide et/ou au moins un coupleur additionnel choisi parmi les méta-phénylènediamines, les méta-aminophénols, les métadiphénols, et leurs sels d'addition avec un acide.

**32.** Composition selon l'une quelconque des revendications précédentes, **caractérisée par le fait que** les sels d'addition avec un acidé des bases d'oxydation et/ou des coupleurs sont choisis parmi les chlorhydrates, les bromhydrates, les sulfates et les tartrates, les lactates et les acétates.

**33.** Composition selon l'une quelconque des revendications précédentes, **caractérisée par le fait que** le milieu approprié pour la teinture est constitué par de l'eau ou par un mélange d'eau et d'au moins un solvant organique.

**34.** Composition selon l'une quelconque des revendications précédentes, **caractérisée par le fait qu'**elle présente un pH compris 5 et 11.

**35.** Composition selon l'une quelconque des revendications précédentes, **caractérisée par le fait qu'**elle contient au moins une peroxydase.

**36.** Procédé de teinture des fibres kératiniques et en particulier des fibres kératiniques humaines telles que les cheveux, **caractérisé par le fait qu'**on applique sur lesdites fibres au moins une composition tinctoriale prête à l'emploi telle que définie dans l'une quelconque des revendications précédentes, pendant un temps suffisant pour développer la coloration désirée.

**37.** Procédé selon la revendication 36, **caractérisé par le fait qu'**il comporte une étape préliminaire consistant à stocker sous forme séparée, d'une part, une composition (A) comprenant, dans un milieu approprié pour la teinture, au moins une base d'oxydation hétérocyclique telle que définie dans l'une quelconque des revendications 1, 8 à 11 et 32 et/ou au moins un coupleur hétérocyclique tel que défini dans l'une quelconque des revendications 1, 14 à 28 et 32 et, d'autre part, une composition (B) renfermant, dans un milieu approprié pour la teinture, au moins

une enzyme de type oxydo-réductase à 2 électrons en présence d'au moins un donneur pour ladite enzyme, puis à procéder à leur mélange au moment de l'emploi avant d'appliquer ce mélange sur les fibres kératiniques.

## Claims

1. Ready-to-use composition for the oxidation dyeing of keratin fibres, and in particular human keratin fibres such as the hair, comprising the combination of at least one oxidation base and at least one coupler, **characterized in that** it comprises, in a medium which is suitable for dyeing, at least one enzyme of 2-electron oxidoreductase type in the presence of at least one donor for the said enzyme, and **in that** the said oxidation base and/or the said coupler is chosen from heterocyclic compounds with the exclusion of monocyclic pyridine compounds.

2. Composition according to Claim 1, **characterized in that** the 2-electron oxidoreductase is chosen from uricases of animal, microbiological or biotechnological origin.

3. Composition according to Claim 1 or 2, **characterized in that** the 2-electron oxidoreductase(s) represent(s) from 0.01 to 20% by weight relative to the total weight of the ready-to-use dye composition.

4. Composition according to Claim 3, **characterized in that** the 2-electron oxidoreductase(s) represent(s) from 0.1 to 5% by weight relative to the total weight of the ready-to-use dye composition.

5. Composition according to Claim 2, **characterized in that** the donor (or substrate) for the said 2-electron oxidoreductase is chosen from uric acid and its salts.

6. Composition according to any one of the preceding claims, **characterized in that** the donor(s) represent(s) from 0.01 to 20% by weight relative to the total weight of the ready-to-use dye composition.

7. Composition according to Claim 6, **characterized in that** the donor(s) represent(s) from 0.1 to 5% by weight relative to the total weight of the ready-to-use dye composition.

8. Composition according to any one of the preceding claims, **characterized in that** the heterocyclic oxidation base (s) is (are) chosen from pyrimidine derivatives and pyrazole derivatives, and the addition salts thereof with an acid.

9. Composition according to Claim 8, **characterized in that** the pyrimidine derivatives are chosen from 2,4,5,6-tetraaminopyrimidine, 4-hydroxy-2,5,6-triaminopyrimidine and pyrazolopyrimidine derivatives, and the addition salts thereof with an acid.

10. Composition according to Claim 9, **characterized in that** the pyrazolopyrimidine derivatives are chosen from pyrazolo[1,5-a]pyrimidine-3,7-diamine, 2-methylpyrazolo[1,5-a]pyrimidine-3,7-diamine, 2,5-dimethylpyrazolo[1,5-a] pyrimidine-3,7-diamine, pyrazolo[1,5-a]pyrimidine-3,5-diamine, 2,7-dimethylpyrazolo[1,5-a]pyrimidine-3,5-diamine, 3-aminopyrazolo[1,5-a]pyrimidin-7-ol, 3-amino-5-methylpyrazolo[1,5-a]pyrimidin-7-ol, 3-aminopyrazolo [1,5-a]pyrimidin-5-ol, 2-(3-aminopyrazolo[1,5-a]pyrimidin-7-ylamino)ethanol, 3-amino-7-β-hydroxyethylamino-5-methylpyrazolo[1,5-a]-pyrimidine, 2-(7-aminopyrazolo[1,5-a]pyrimidin-3-ylamino)ethanol, 2-[(3-aminopyrazolo [1,5-a]pyrimidin-7-yl)(2-hydroxyethyl)amino]ethanol, 2-[(7-aminopyrazolo[1,5-a]pyrimidin-3-yl)(2-hydroxyethyl) amino]ethanol, 5,6-dimethylpyrazolo[1,5-a]pyrimidine-3,7-diamine, 2,6-dimethylpyrazolo[1,5-a]pyrimidine-3,7-diamine and 2,5,N-7,N-7-tetramethylpyrazolo[1,5-a]pyrimidine-3,7-diamine, and the addition salts thereof and the tautomers thereof, when a tautomeric equilibrium exists.

11. Composition according to Claim 8, **characterized in that** the pyrazole derivatives are chosen from 4,5-diaminopyrazole, 4,5-diamino-1-methylpyrazole, 1-benzyl-4,5-diaminopyrazole, 3,4-diaminopyrazole, 1-benzyl-4,5-diamino-3-methylpyrazole, 4-amino-1,3-dimethyl-5-hydrazinopyrazole, 4,5-diamino-3-methyl-1-phenylpyrazole, 4,5-diamino-3-methyl-1-tert-butylpyrazole, 4,5-diamino-1-methyl-3-tert-butylpyrazole, 4,5-diamino-1-ethyl-3-methylpyrazole, 4,5-diamino-1-ethyl-3-(4'-methoxyphenyl)pyrazole, 4,5-diamino-1-ethyl-3-hydroxymethylpyrazole, 4,5-diamino-3-hydroxymethyl-1-methylpyrazole, 4,5-diamino-3-hydroxymethyl-1-isopropylpyrazole and 4,5-diamino-3-methyl-1-isopropylpyrazole, and the addition salts thereof with an acid.

12. Composition according to any one of the preceding claims, **characterized in that** the heterocyclic oxidation base (s) represent(s) from 0.0005 to 12% by weight relative to the total weight of the ready-to-use dye composition.

**13.** Composition according to Claim 12, **characterized in that** the heterocyclic oxidation base(s) represent(s) from 0.005 to 6% by weight relative to the total weight of the ready-to-use dye composition.

**14.** Composition according to any one of the preceding claims, **characterized in that** the heterocyclic coupler(s) is (are) chosen from indole derivatives, indoline derivatives, benzimidazole derivatives, benzomorpholine derivatives, sesamol derivatives, pyrazoloazole derivatives, pyrroloazole derivatives, imidazoloazole derivatives, pyrazolopyrimidine derivatives, pyrazoline-3,5-dione derivatives, pyrrolo[3,2-d]oxazole derivatives, pyrazolo[3,4-d]thiazole derivatives, thiazoloazole S-oxide derivatives and thiazoloazole S,S-dioxide derivatives, and the addition salts thereof with an acid.

**15.** Composition according to Claim 14, **characterized in that** the indole derivatives are chosen from the compounds of formula (I) below, and the addition salts thereof with an acid:

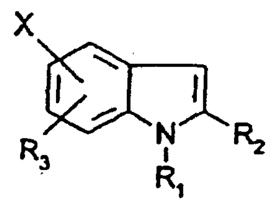

(I)

in which:

$R_1$ represents a hydrogen atom, a $C_1$-$C_4$ alkyl radical, a $C_2$-$C_4$ mono- or polyhydroxyalkyl radical or a $C_1$-$C_4$ aminoalkyl radical in which the amine is mono- or disubstituted with a $C_1$-$C_4$ alkyl group;
$R_2$ represents a hydrogen atom or a $C_1$-$C_4$ alkyl radical;
$R_3$ represents a hydrogen atom or a $C_1$-$C_4$ alkyl or hydroxyl radical;
X represents a hydroxyl radical or a radical $NHR_4$ in which $R_4$ represents a hydrogen atom or a $C_1$-$C_4$ alkyl or $C_1$-$C_4$ hydroxyalkyl radical.

**16.** Composition according to Claim 15, **characterized in that** the indole derivatives are chosen from 4-hydroxyindole, 6-hydroxyindole, 7-aminoindole, 6-aminoindole, 7-hydroxyindole, 7-ethyl-6-(β-hydroxyethyl)aminoindole, 4-aminoindole, 6-hydroxy-1-methylindole, 5,6-dihydroxyindole, 4-hydroxy-1-N-methylindole, 4-hydroxy-2-methylindole, 4-hydroxy-5-methylindole, 4-hydroxy-1-N-(β-hydroxyethyl)indole, 4-hydroxy-1-N-(β-hydroxypropyl)indole, 1-N-(β,γ-dihydroxypropyl)-4-hydroxyindole, 4-hydroxy-1-N-(β-hydroxyethyl)-5-methylindole and 1-N-(γ-dimethylaminopropyl)-4-hydroxyindole, and the addition salts thereof with an acid.

**17.** Composition according to Claim 14, **characterized in that** the indoline derivatives are chosen from 4-hydroxyindoline, 6-hydroxyindoline, 6-aminoindoline and 5,6-dihydroxyindoline, and the addition salts thereof with an acid.

**18.** Composition according to Claim 14, **characterized in that** the benzimidazole derivatives are chosen from the compounds of formula (II) below, and the addition salts thereof with an acid:

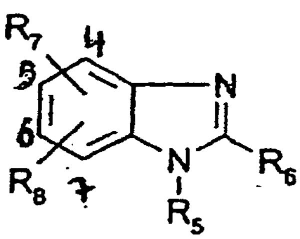

(II)

in which:

$R_5$ represents a hydrogen atom or a $C_1$-$C_4$ alkyl radical,
$R_6$ represents a hydrogen atom or a $C_1$-$C_4$ alkyl or phenyl radical,

$R_7$ represents a hydroxyl, amino or methoxy radical,
$R_8$ represents a hydrogen atom or a hydroxyl, methoxy or $C_1$-$C_4$ alkyl radical;

with the proviso that:

- when $R_7$ denotes an amino radical, then it occupies position 4,
- when $R_7$ occupies position 4, then $R_8$ occupies position 7,
- when $R_7$ occupies position 5, then $R_8$ occupies position 6.

**19.** Composition according to Claim 18, **characterized in that** the benzimidazole derivatives are chosen from 4-hydroxybenzimidazole, 4-aminobenzimidazole, 4-hydroxy-7-methylbenzimidazole, 4-hydroxy-2-methylbenzimidazole, 1-butyl-4-hydroxybenzimidazole, 4-amino-2-methylbenzimidazole, 5,6-dihydroxybenzimidazole, 5-hydroxy-6-methoxybenzimidazole, 4,7-dihydroxybenzimidazole, 4,7-dihydroxy-1-methylbenzimidazole, 4,7-dimethoxybenzimidazole, 5,6-dihydroxy-1-methylbenzimidazole, 5,6-dihydroxy-2-methylbenzimidazole and 5,6-dimethoxybenzimidazole, and the addition salts thereof with an acid.

**20.** Composition according to Claim 14, **characterized in that** the benzomorpholine derivatives are chosen from the compounds of formula (III) below, and the addition salts thereof with an acid:

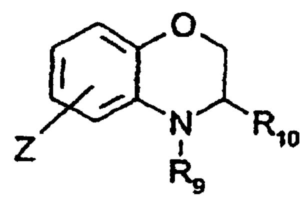

(III)

in which:

$R_9$ and $R_{10}$, which may be identical or different, represent a hydrogen atom or a $C_1$-$C_4$ alkyl radical,
Z represents a hydroxyl or amino radical.

**21.** Composition according to Claim 20, **characterized in that** the benzomorpholine derivatives are chosen from 6-hydroxy-1,4-benzomorpholine, N-methyl-6-hydroxy-1,4-benzomorpholine and 6-amino-1,4-benzomorpholine, and the addition salts thereof with an acid.

**22.** Composition according to Claim 14, **characterized in that** the sesamol derivatives are chosen from the compounds of formula (IV) below; and the addition salts thereof with an acid:

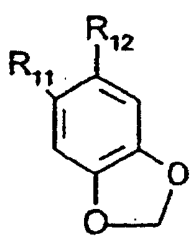

(IV)

in which:

$R_{11}$ denotes a hydroxyl, amino, ($C_1$-$C_4$)alkylamino, monohydroxy($C_1$-$C_4$) alkylamino or polyhydroxy($C_2$-$C_4$) alkylamino radical,
$R_{12}$ denotes a hydrogen or halogen atom or a $C_1$-$C_4$ alkoxy radical.

**23.** Composition according to Claim 22, **characterized in that** the sesamol derivatives are chosen from 2-bromo-4,5-methylenedioxyphenol, 2-methoxy-4,5-methylenedioxyaniline and 2-(β-hydroxyethyl)amino-4,5-methylenedioxybenzene, and the addition salts thereof with an acid.

**24.** Composition according to Claim 14, **characterized in that** the pyrazoloazole derivatives are chosen from:

- 2-methylpyrazolo[1,5-b]-1,2,4-triazole,
- 2-ethylpyrazolo[1,5-b]-1,2,4-triazole,
- 2-isopropylpyrazolo[1,5-b]-1,2,4-triazole,
- 2-phenylpyrazolo[l,5-b]-1,2,4-triazole,
- 2,6-dimethylpyrazolo[l,5-b]-1,2,4-triazole,
- 7-chloro-2,6-dimethylpyrazolo[1,5-b]-1,2,4-triazole,
- 3,6-dimethylpyrazolo[3,2-c]-1,2,4-triazole,
- 6-phenyl-3-methylthiopyrazolo[3,2-c]-1,2,4-triazole and
- 6-aminopyrazolo[1,5-a]benzimidazole,

and the addition salts thereof with an acid.

**25.** Composition according to Claim 14, **characterized in that** the pyrroloazole derivatives are chosen from:

- 5-cyano-4-ethoxycarbonyl-8-methylpyrrolo[1,2-b]-1,2,4-triazole,
- 5-cyano-8-methyl-4-phenylpyrrolo[1,2-b]-1,2,4-triazole,
- 7-amido-6-ethoxycarbonylpyrrolo[1,2-a]-benzimidazole,

and the addition salts thereof with an acid.

**26.** Composition according to Claim 14, **characterized in that** the imidazoloazole derivatives are chosen from:

- 7,8-dicyanoimidazolo[3,2-a]imidazole,
- 7,8-dicyano-4-methylimidazolo[3,2-a]imidazole,

and the addition salts thereof with an acid.

**27.** Composition according to Claim 14, **characterized in that** the pyrazolopyrimidine derivatives are chosen from:

- pyrazolo[1,5-a]pyrimidin-7-one,
- 2,5-dimethylpyrazolo[1,5-a]pyrimidin-7-one,
- 2-methyl-6-ethoxycarbonylpyrazolo[1,5-a]pyrimidin-7-one,
- 2-methyl-5-methoxymethylpyrazolo[1,5-a]pyrimidin-7-one,
- 2-tert-butyl-5-trifluoromethylpyrazolo[1,5-a]-pyrimidin-7-one,
- 2,7-dimethylpyrazolo[1,5-a]pyrimidin-5-one,

and the addition salts thereof with an acid.

**28.** Composition according to Claim 14, **characterized in that** the pyrazoline-3,5-dione derivatives are chosen from:

- 1,2-diphenylpyrazoline-3,5-dione,
- 1,2-diethylpyrazoline-3,5-dione,

and the addition salts thereof with an acid.

**29.** Composition according to any one of the preceding claims, **characterized in that** the heterocyclic coupler(s) represent(s) from 0.0001 to 10% by weight relative to the total weight of the ready-to-use dye composition.

**30.** Composition according to Claim 29, **characterized in that** the heterocyclic coupler(s) represent(s) from 0.005 to 5% by weight relative to the total weight of the ready-to-use dye composition.

**31.** Composition according to any one of the preceding claims, **characterized in that** it contains at least one additional oxidation base chosen from para-phenylenediamines, bis(phehyl)alkylenediamines, ortho-phenylenediamines, para-aminophenols and ortho-aminophenols, and the addition salts thereof with an acid and/or at least one additional coupler chosen from meta-phenylenediamines, meta-aminophenols and meta-diphenols, and the addition salts thereof with an acid.

**32.** Composition according to any one of the preceding claims, **characterized in that** the addition salts with an acid of the oxidation bases and/or couplers are chosen from the hydrochlorides, hydrobromides, sulphates, tartrates, lactates and acetates.

**33.** Composition according to any one of the preceding claims, **characterized in that** the medium which is suitable for dyeing consists of water or a mixture of water and at least one organic solvent.

**34.** Composition according to any one of the preceding claims, **characterized in that** it has a pH of between 5 and 11.

**35.** Composition according to any one of the preceding claims, **characterized in that** it contains at least one peroxidase.

**36.** Process for dyeing keratin fibres, and in particular human keratin fibres such as the hair, **characterized in that** at least one ready-to-use dye composition as defined in any one of the preceding claims is applied to the said fibres, for a period which is sufficient to develop the desired coloration.

**37.** Process according to Claim 36, **characterized in that** it includes a preliminary step which consists in separately storing, on the one hand, a composition (A) comprising, in a medium which is suitable for dyeing, at least one heterocyclic oxidation base as defined in any one of Claims 1, 8 to 11 and 32 and/or at least one heterocyclic coupler as defined in any one of Claims 1, 14 to 28 and 32, and, on the other hand, a composition (B) comprising, in a medium which is suitable for dyeing, at least one enzyme of 2-electron oxidoreductase type in the presence of at least one donor for the said enzyme, and then in mixing them together at the time of use, after which this mixture is applied to the keratin fibres.

**Patentansprüche**

**1.** Gebrauchsfertige Zusammensetzung zum oxidativen Färben von Keratinfasern und insbesondere menschlichen Keratinfasern, wie dem Haar, die mindestens eine Oxidationsbase in Kombination mit mindestens einem Kuppler enthält, **dadurch gekennzeichnet, dass** sie in einem zum Färben geeigneten Medium mindestens ein Enzym vom Typ der Oxidoreduktasen (2 Elektronen) in Gegenwart mindestens eines Donors für das Enzym enthält und dadurch, dass die Oxidationsbase und/oder der Kuppler unter den heterocyclischen Verbindungen ausgewählt sind, wobei monocyclische Pyridinderivate ausgenommen sind.

**2.** Zusammensetzung nach Anspruch 1, **dadurch gekennzeichnet, dass** die Oxidoreduktasen (2 Elektronen) unter den Uricasen tierischer, mikrobiologischer oder biotechnologischer Herkunft ausgewählt sind.

**3.** Zusammensetzung nach einem der Ansprüche 1 oder 2, **dadurch gekennzeichnet, dass** die Oxidoreduktasen (2 Elektronen) 0,01 bis 20 Gew.-% des Gesarntgewichts der gebrauchsfertigen Farbmittelzusammensetzung ausmachen.

**4.** Zusammensetzung nach Anspruch 3, **dadurch gekennzeichnet, dass** die Oxidoreduktasen (2 Elektronen) 0,1 bis 5 Gew.-% des Gesamtgewichts der gebrauchsfertigen Farbmittelzusammensetzung ausmachen.

**5.** Zusammensetzung nach Anspruch 2, **dadurch gekennzeichnet, dass** der Donor (oder das Substrat) für die Oxidoreduktasen (2 Elektronen) unter Harnsäure und ihren Salzen ausgewählt ist.

**6.** Zusammensetzung nach einem der vorhergehenden Ansprüche, **dadurch gekennzeichnet, dass** der Donor oder die Donoren 0,01 bis 20 Gew.-% des Gesamtgewichts der gebrauchsfertigen Farbmittel-Zusammensetzung ausmachen.

**7.** Zusammensetzung nach Anspruch 6, **dadurch gekennzeichnet, dass** der Donor oder die Donoren 0,1 bis 5 Gew.-% des Gesamtgewichts der gebrauchsfertigen Farbmittelzusammensetzung ausmachen.

**8.** Zusammensetzung nach einem der vorhergehenden Ansprüche, **dadurch gekennzeichnet, dass** die heterocyclische(n) Oxidationsbase(n) unter den Pyrimidinderivate, Pyrazolderivaten und deren Additionssalzen mit einer Säure ausgewählt sind.

**9.** Zusammensetzung nach Anspruch 8,**dadurch gekennzeichnet, dass** die Pyrimidinderivate unter 2,4,5,6-Tetraaminopyrimidin, 4-Hydroxy-2,5,6-triaminopyrimidin, Pyrazolo-pyrmidinderivaten und deren Additionssalzen mit einer Säure ausgewählt sind.

**10.** Zusammensetzung nach Anspruch 9, **dadurch gekennzeichnet, dass** die Pyrazolo-pyrimidinderivate unter Pyrazolo-[1,5-a]-pyrimidin-3,7-diamin, 2-Methyl-pyrazolo-[1,5-a]-pyrimidin-3,7-diamin, 2,5-Dimethyl-pyrazolo-[1,5-a]-pyrimidin-3,7-diamin, Pyrazolo-[1,5-a]-pyrimidin-3,5-diamin, 2,7-Dimethyl-pyrazolo-[1,5-a]-pyrimidin-3,5-diamin, 3-Amino-pyrazolo-[1,5-a]-pyrimidin-7-ol, 3-Amino-5-methyl-pyrazolo-[1,5-a]-pyrimidin-7-ol, 3-Amino-pyrazolo-[1,5-a]-pyrimidin-5-ol, 2-(3-Amino-pyrazolo-[1,5-a]-pyrimidin-7-ylamino)-ethanol, 3-Amino-7-β-hydroxyethylamino-5-methylpyrazolo-[1,5-a]-pyrimidin, 2-(7-Amino-pyrazolo-[1,5-a]-pyrimidin-3-ylamino)-ethanol, 2-[(3-Amino-pyrazolo-[1,5-a]-pyrimidin-7-yl)-(2-hydroxyethyl)amino]-ethanol, 2-[(7-Amino-pyrazolo-[1,5-a]-pyrimidin-3-yl)-(2-hydroxyethyl)amino]-ethanol, 5,6-Dimethylpyrazolo-[1,5-a]-pyrimidin-3,7-diamin, 2,6-Dimethyl-pyrazolo-[1,5-a]-pyrimidin-3,7-diamin, und 2,5,N7,N7-Tetramethyl-pyrazolo-[1,5-a]-pyrimidin-3,7-diamin und deren Additionssalzen und deren tautomeren Formen, wenn tautomere Formen im Gleichgewicht vorliegen, ausgewählt sind.

**11.** Zusammensetzung nach Anspruch 8, **dadurch gekennzeichnet, dass** die Pyrazolderivate unter 4,5-Diamino-pyrazol, 4,5-Diamino-1-methyl-pyrazol, 1-Benzyl-4,5-diamino-pyrazol, 3,4-Diamino-pyrazol, 1-Benzyl-4,5-diamino-3-methyl-pyrazol, 4-Amino-1,3-dimethyl-5-hydrazino-pyrazol, 4,5-Diamino-3-methyl-1-phenylpyrazol, 4,5-Diamino-3-methyl-1-*t*-butyl-pyrazol, 4,5-Diamino-1-methyl-3-*t*-butyl-pyrazol, 4,5-Diamino-1-ethyl-3-methyl-pyrazol, 4,5-Diamino-1-ethyl-3-(4'-methoxyphenyl)-pyrazol, 4,5-Diamino-1-ethyl-3-hydroxymethyl-pyrazol, 4,5-Diamino-3-hydroxymethyl-1-methyl-pyrazol, 4,5-Diamino-3-hydroxymethyl-1-isopropyl-pyrazol, 4,5-Diamino-3-methyl-1-isopropyl-pyrazol und deren Additionssalzen mit einer Säure ausgewählt sind.

**12.** Zusammensetzung nach einem der vorhergehenden Ansprüche, **dadurch gekennzeichnet, dass** die heterocyclische(n) Oxidationsbase(n) 0,0005 bis 12 Gew.-% des Gesamtgewichts der gebrauchsfertigen Farbmittelzusammensetzung ausmachen.

**13.** Zusammensetzung nach Anspruch 12, **dadurch gekennzeichnet, dass** die heterocyclische(n) Oxidationsbase (n) 0,005 bis 6 Gew.-% des Gesamtgewichts der gebrauchsfertigen Farbmittelzusammensetzung ausmachen.

**14.** Zusammensetzung nach einem der vorhergehenden Ansprüche, **dadurch gekennzeichnet, dass** der oder die heterocyclische(n) Kuppler unter den Indolderivaten, Indolinderivaten, Benzimidazolderivaten, Benzomorpholinderivaten, Sesamoldenvaten, Pyrazoloazolderivaten, Pyrrolo-azolderivaten, Imidazolo-azolderivaten, Pyrazolopyrimidinderivaten, Pyrazolin-3,5-dionen, Pyrrolo-[3,2-d]-oxazolderivaten, Pyrazolo-[3,4-d]-thiazolderiaten, S-Oxid-thiazoloazolderivaten, S,S-Dioxid-thiazolo-azolderivaten und deren Additionssalzen mit einer Säure ausgewählt sind.

**15.** Zusammensetzung nach Anspruch 14, **dadurch gekennzeichnet, dass** die Indolderivate unter den Verbindungen der folgenden Formel (I) und ihren Additionssalzen mit einer Säure ausgewählt sind:

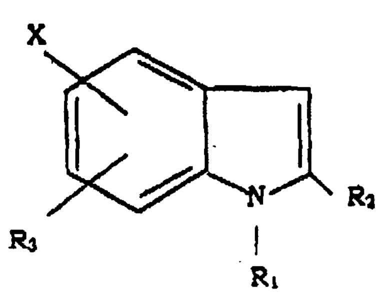

(I)

worin bedeuten:

- $R_1$ Wasserstoff, $C_{1-4}$-Alkyl, $C_{2-4}$-Monohydroxyalkyl, $C_{2-4}$-Polyhydroxyalkyl oder $C_{1-4}$-Aminoalkyl, deren Aminogruppe mit einer $C_{1-4}$-Alkylgruppe mono- oder disubstituiert ist;
- $R_2$ Wasserstoff oder $C_{1-4}$-Alkyl;
- $R_3$ Wasserstoff, $C_{1-4}$-Alkyl oder Hydroxy;
- X Hydroxy oder $NHR_4$, wobei $R_4$ Wasserstoff, $C_{1-4}$-Alkyl oder $C_{1-4}$-Hydroxyalkyl bedeutet.

**16.** Zusammensetzung nach Anspruch 15, **dadurch gekennzeichnet, dass** die Indolderivate unter 4-Hydroxyindol, 6-Hydroxyindol, 7-Aminoindol, 6-Aminoindol, 7-Hydroxyindol, 7-Ethyl-6-(β-hydroxyethyl)amino-indol, 4-Aminoindol, 6-Hydroxy-1-methylindol, 5,6-Dihydroxyindol, 4-Hydroxy-1-N-methylindol, 4-Hydroxy-2-methylindol, 4-Hydroxy-5-methylindol, 4-Hydroxy-1-N-(β-hydroxyethyl)-indol, 4-Hydroxy-1-N-(β-hydroxypropyl)-indol, 1-N-(β,γ-Dihydroxypropyl)-4-hydroxy-indol, 4-Hydroxy-1-N-(β-hydroxyethyl)-5-methylindol, 1-N-(γ-Dimethylaminopropyl)-4-hydroxy-indol und deren Additionssalzen mit einer Säure ausgewählt sind.

**17.** Zusammensetzung nach Anspruch 14, **dadurch gekennzeichnet, dass** die Indolinderivate unter 4-Hydroxyindolin, 6-Hydroxyindolin, 6-Aminoindolin, 5,6-Dihydroxymdolin und deren Additionssalzen mit einer Säure ausgewählt sind.

**18.** Zusammensetzung nach Anspruch 14, **dadurch gekennzeichnet, dass** die Benzimidazolderivate unter den Verbindungen der folgenden Formel (II) und ihren Additionssalzen mit einer Säure ausgewählt sind:

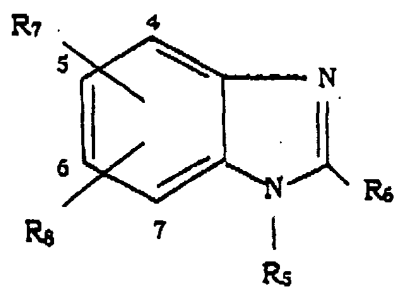

(II)

worin bedeuten:

- $R_5$ Wasserstoff oder $C_{1-4}$-Alkyl;
- $R_6$ Wasserstoff, $C_{1-4}$-Alkyl oder Phenyl;
- $R_7$ Hydroxy, Amino oder Methoxy;
- $R_8$ Wasserstoff, Hydroxy, Methoxy oder $C_{1-4}$-Alkyl;

mit der Maßgabe, dass

- sich die Gruppe $R_7$ in 4-Stellung befindet, wenn $R_7$ Amino bedeutet;
- sich die Gruppe $R_8$ in 7-Stellung befindet, wenn sich die Gruppe $R_7$ in 4-Stellung befindet, und
- sich die Gruppe $R_8$ in 6-Stellung befindet, wenn sich die Gruppe $R_7$ in 5-Stellung befindet.

**19.** Zusammensetzung nach Anspruch 18, **dadurch gekennzeichnet, dass** die Benzimidazolderivate unter 4-Hydroxy-benzimidazol, 4-Amino-benzimidazol, 4-Hydroxy-7-methyl-benzimidazol, 4-Hydroxy-2-methyl-benzimidazol, 1-Butyl-4-hydroxy-benzimidazol, 4-Amino-2-methyl-benzimidazol, 5,6-Dihydroxy-benzimidazol, 5-Hydroxy-6-methoxy-benzimidazol, 4,7-Dihydroxy-benzimidazol, 4,7-Dihydroxy-1-methyl-benzimidazol, 4,7-Dimethoxy-benzimidazol, 5,6-Dihydroxy-1-methyl-benzimidazol, 5,6-Dihydroxy-2-methylbenzimidazol, 5,6-Dimethoxy-benzimidazol und deren Additionssalzen mit einer Säure ausgewählt sind.

**20.** Zusammensetzung nach Anspruch 14, **dadurch gekennzeichnet, dass** die Benzomorpholinderivate unter den Verbindungen der folgenden Formel (III) und ihren Additionssalzen mit einer Säure ausgewählt sind:

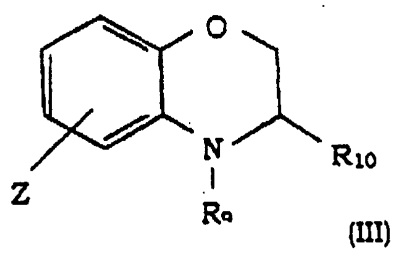

(III)

worin bedeuten:

- $R_9$ und $R_{10}$, die gleich oder verschieden sind, Wasserstoff oder $C_{1-4}$-Alkyl;
- Z Hydroxy oder Amino.

**21.** Zusammensetzung nach Anspruch 20, **dadurch gekennzeichnet, dass** die Benzomorpholinderivate unter 6-Hydroxy-1,4-benzomorpholin, N-Methyl-6-hydroxy-1,4,-benzomorpholin, 6-Amino-1,4-benzomorpholin und deren Additionssalzen mit einer Säure ausgewählt sind.

**22.** Zusammensetzung nach Anspruch 14, **dadurch gekennzeichnet, dass** die Sesamolderivate unter den Verbindungen der folgenden Formel (IV) und ihren Additionssalzen mit einer Säure ausgewählt sind:

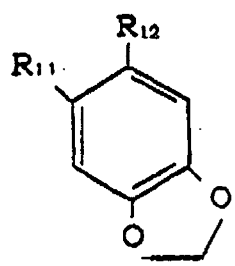

(IV)

worin bedeuten:

- $R_{11}$ Hydroxy, Amino, $C_{1-4}$-Alkylamino, $C_{1-4}$-Monohydroxyalkylamino oder $C_{2-4}$-Polyhydroxyalkylamino;
- $R_{12}$ Wasserstoff, Halogen oder $C_{1-4}$-Alkoxy.

**23.** Zusammensetzung nach Anspruch 22, **dadurch gekennzeichnet, dass** die Sesamolderivate unter 2-Brom-4,5-methylendioxy-phenol, 2-Methoxy-4,5-methylendioxy-anilin, 2-(β-Hydroxyethyl)amino-4,5-methylendioxy-benzol und deren Additionssalzen mit einer Säure ausgewählt sind.

**24.** Zusammensetzung nach Anspruch 14, **dadurch gekennzeichnet, dass** die Pyrazolo-azolderivate ausgewählt sind unter:

- 2-Methyl-pyrazolo-[1,5-b]-1,2,4-triazol,
- 2-Ethyl-pyrazolo-[1,5-b]-1,2,4-triazol,
- 2-Isopropyl-pyrazolo-[1,5-b]-1,2,4-triazol,
- 2-Phenyl-pyrazolo-[1,5-b]-1,2,4-triazol,
- 2,6-Dimethyl-pyrazolo-[1,5-b]-1,2,4-triazol,
- 7-Chlor-2,6-dimethyl-pyrazolo-[1,5-b]-1,2,4-triazol,
- 3,6-Dimethyl-pyrazolo-[3,2-c]-1,2,4-triazol,
- 6-Phenyl-3-methylthio-pyrazolo-[3,2-c]-1,2,4-triazol,
- 6-Amino-pyrazolo-[1,5-a]-benzimidazol,

und deren Additionssalzen mit einer Säure

**25.** Zusammensetzung nach Anspruch 14, **dadurch gekennzeichnet, dass** die Pyrrolo-azolderivaten ausgewählt

sind unter:

- 5-Cyano-4-ethoxycarbonyl-8-methyl-pyrrolo-[1,2-b]-1,2,4-triazol,
- 5-Cyano-8-methyl-4-phenyl-pyrrolo-[1,2-b]-1,2,4-triazol,
- 7-Amido-6-ethoxycarbonyl-pyrrolo-[1,2-a]-benzimidazol,
- und deren Additionssalzen mit einer Säure.

**26.** Zusammensetzung nach Anspruch 14, **dadurch gekennzeichnet, dass** die Imidazolo-azolderivaten ausgewählt sind unter:

- 7,8-Dicyano-imidazolo-[3,2-a]-imidazol,
- 7,8-Dicyano-4-methyl-imidazolo-[3,2-a]-imidazol,
- und deren Additionssalzen mit einer Säure.

**27.** Zusammensetzung nach Anspruch 14, **dadurch gekennzeichnet, dass** die Pyrazolo-pyrimidinderivate ausgewählt sind unter;

- Pyrazolo-[1,5-a]-pyrimidin-7-on,
- 2,5-Dimethyl-pyrazolo-[1,5-a]-pyrimidin-7-on,
- 2-Methyl-6-ethoxycarbonyl-pyrazolo-[1,5-a]-pyrimidin-7-on,
- 2-Methyl-5-methoxymethyl-pyrazolo-[1,5-a]-pyrimidin-7-on,
- 2-t-Butyl-5-trifluormethyl-pyrazolo-[1,5-a]-pyrimidin-7-on,
- 2,7-Dimethyl-pyrazolo-[1,5-a]-Pyrimidin-5-on,
- und deren Additionssalzen mit einer Säure.

**28.** Zusammensetzung nach Anspruch 14, **dadurch gekennzeichnet, dass** die Pyrazolin-3,5-dionderivateausgewählt sind unter:

- 1,2-Diphenyl-pyrazolin-3,5-dion;
- 1,2-Diethyl-pyrazolin-3,5-dion;
- und deren Additionssalzen mit einer Säure.

**29.** Zusammensetzung nach einem der vorhergehenden Ansprüche, **dadurch gekennzeichnet, dass** der oder die heterocyclische(n) Kuppler 0,0001 bis 10 Gew.-% des Gesamtgewichts der gebrauchsfertigen Farbmittelzusammensetzung ausmachen.

**30.** Zusammensetzung nach Anspruch 29, **dadurch gekennzeichnet, dass** der oder die heterocyclische(n) Kuppler 0,005 bis 5 Gew.-% des Gesamtgewichts der gebrauchsfertigen Farbmittelzusammensetzung ausmachen.

**31.** Zusammensetzung nach einem der vorhergehenden Ansprüche, **dadurch gekennzeichnet, dass** sie ferner mindestens eine zusätzliche Oxidationsbase, die unter den p-Phenylendiaminen, Bisphenylalkylendiaminen, o-Phenylendiaminen, o-Aminophenolen, p-Aminophenolen und ihren Additionssalzen mit einer Säure ausgewählt ist, und/oder mindestens einen zusätzlichen Kuppler enthält, der unter den m-Phenylendiaminen, m-Aminophenolen, m-Dihydroxybenzolen und ihren Additionssalzen mit einer Säure ausgewählt ist.

**32.** Zusammensetzung nach einem der vorhergehenden Ansprüche, **dadurch gekennzeichnet, dass** die Additionssalze der Oxidationsbasen und der Kuppler mit einer Säure unter den Hydrochloriden, Hydrobromiden, Sulfaten, Tartraten, Lactaten und Acetaten ausgewählt sind.

**33.** Zusammensetzung nach einem der vorhergehenden Ansprüche, **dadurch gekennzeichnet, dass** das zum Färben geeignete Medium aus Wasser oder einem Gemisch von Wasser und mindestens einem organischen Lösungsmittel besteht.

**34.** Zusammensetzung nach einem der vorhergehenden Ansprüche, **dadurch gekennzeichnet, dass** sie einen pH-Wert im Bereich von 5 bis 11 aufweist.

**35.** Zusammensetzung nach einem der vorhergehenden Ansprüche, **dadurch gekennzeichnet, dass** sie mindestens eine Peroxidase enthält.

**36.** Verfahren zum Färben von Keratinfasern und insbesondere menschlichen Keratinfasern, wie dem Haar, **dadurch gekennzeichnet, dass** auf die Fasern während einer Zeitspanne, die zur Entwicklung der gewünschten Färbung ausreichend ist, mindestens eine gebrauchsfertige Farbmittelzusammensetzung nach einem der vorhergehenden Ansprüche aufgebracht wird.

**37.** Verfahren nach Anspruch 36, **dadurch gekennzeichnet, dass** es einen vorbereitenden Schritt umfasst, der darin besteht, einerseits eine Zusammensetzung (A), die in einem zum Färben geeigneten Medium mindestens eine heterocyclische Oxidationsbase nach einem der Ansprüche 1, 8 bis 11 und 32 und/oder mindestens einen heterocyclischen Kuppler nach einem der Ansprüche 1, 14 bis 28 und 32 enthält, und andererseits eine Zusammensetzung (B) getrennt voneinander aufzubewahren, die in einem zum Färben geeigneten Medium mindestens ein Enzym vom Typ Oxidoreduktase (2 Elektronen) in Gegenwart mindestens eines Donors für das Enzym enthält, und diese bei der Anwendung zu vermischen, bevor das Gemisch auf die Fasern aufgebracht wird.